(19) Europäisches Patentamt / European Patent Office / Office européen des brevets

(11) EP 2 746 302 A1

(12) EUROPEAN PATENT APPLICATION
published in accordance with Art. 153(4) EPC

(43) Date of publication:
**25.06.2014 Bulletin 2014/26**

(21) Application number: **12824078.5**

(22) Date of filing: **31.07.2012**

(51) Int Cl.:
*C08F 10/14* (2006.01)
*C07C 2/24* (2006.01)
*C07C 11/02* (2006.01)
*C07F 17/00* (2006.01)
*C08F 4/6592* (2006.01)

(86) International application number:
**PCT/JP2012/069511**

(87) International publication number:
**WO 2013/024701 (21.02.2013 Gazette 2013/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.08.2011 JP 2011176799**

(71) Applicant: **Idemitsu Kosan Co., Ltd**
**Tokyo 100-8321 (JP)**

(72) Inventors:
- **TSUJI, Minako**
  **Ichihara-shi**
  **Chiba 299-0193 (JP)**
- **OKAMOTO, Takuji**
  **Ichihara-shi**
  **Chiba 299-0193 (JP)**
- **FUJIMURA, Takenori**
  **Ichihara-shi**
  **Chiba 299-0193 (JP)**
- **MINAMI, Yutaka**
  **Ichihara-shi**
  **Chiba 299-0193 (JP)**

(74) Representative: **HOFFMANN EITLE**
**Patent- und Rechtsanwälte**
**Arabellastrasse 4**
**81925 München (DE)**

# (54) OLEFIN OLIGOMER AND PRODUCTION METHOD THEREOF

(57) An α-olefin oligomer having the following features (1) to (6), and a method for producing the α-olefin oligomer using a particular double-crosslinked meso complex provide an α-olefin oligomer that contains a smaller amount of a dimer component while having a low viscosity, not in accordance with the Schulz-Flory distribution, and a method for producing the same:
(1) a trimer component proportion C3 (% by mass) that is larger than a theoretical value obtained from the S-F distribution,
(2) a hexamer component proportion C6 (% by mass) that is smaller than a theoretical value obtained from the S-F distribution,
(3) a kinematic viscosity at 100°C of 20 $mm^2/s$ or less,
(4) a meso triad fraction [mm] measured by $^{13}C$-NMR of 40% by mol of less,
(5) from 0.2 to 1.0 of a vinylidene group per one molecule, and
(6) an average carbon number of a monomer unit of from 6 to 30,

wherein the S-F distribution is calculated based on a chain propagation probability α calculated from a dimer component proportion C2 (% by mass).

EP 2 746 302 A1

## Description

[Technical Field]

The present invention relates to an α-olefin oligomer and a method for producing the same, and more specifically relates to an α-olefin oligomer that is useful as a wax component and a lubricating oil component, and a method for producing the same.

[Background Art]

In recent years, an α-olefin oligomer is produced with a metallocene catalyst and is used as a wax component and a lubricating oil component. For example, Patent Document 1 discloses a polymer obtained by polymerizing a higher α-olefin having 10 or more carbon atoms with a metallocene catalyst, and a catalyst containing a racemic double-crosslinked complex is used in the working examples thereof. Patent Document 2 discloses a method for producing an α-olefin polymer, in which an α-olefin having 4 or more carbon atoms is polymerized with a metallocene catalyst, and a catalyst containing a meso double-crosslinked complex having two different crosslinking groups is used in the working examples thereof.

An α-olefin oligomer has been produced with a metallocene catalyst as described above, but the selectivity of products in the system using the ordinary metallocene catalyst is in accordance with the certain rule (Schulz-Flory distribution, see Non-patent Documents 1 to 3), and thus a large amount of ultralow molecular weight components, particularly a dimer, is formed in production of a low molecular weight product, which disadvantageously results in increase of a VOC (volatile organic compound) component and stickiness due to the dimer component in a solid oligomer. For example, in the case where an α-olefin oligomer is used as a wax component or a lubricating oil component, it is necessary to remove the unnecessary ultralow molecular weight components through distillation, which deteriorates the yield of products. The α-olefin oligomers obtained by the methods disclosed in Patent Documents 1 to 5 are still insufficient in reduction of the dimer component, and further improvement in performance has been demanded.

[Related Art Documents]

[Patent Documents]

Patent Document 1: WO 03/070790
Patent Document 2: JP-A-2001-335607
Patent Document 3: WO 2010/053022
Patent Document 4: WO 2010/117028
Patent Document 5: JP-T-2009-501836

[Non-patent Documents]

Non-patent Document 1: J. Am. Chem. Soc., 1940, 62(6), 1561-1565
Non-patent Document 2: Adv. Polymer. Sci, 1974, 15(1), 1-30
Non-patent Document 3: J. Am. Chem. Soc., 2004, 126, 10701-10712

[Summary of the Invention]

[Problems to be solved by the Invention]

Patent Document 1 discloses a polymer obtained by polymerizing a higher α-olefin having 10 or more carbon atoms with a metallocene catalyst, and a catalyst containing a racemic double-crosslinked complex is used in the working examples thereof. However, in the case where a racemic metallocene catalyst is used, the increase of the reaction temperature lowers the molecular weight, but a low molecular weight product is not obtained due to the high regularity.

Patent Document 2 discloses a method for producing an α-olefin polymer, in which an α-olefin having 4 or more carbon atoms is polymerized with a metallocene catalyst, and a catalyst containing a meso double-crosslinked complex having two different crosslinking groups (C/Si) is used in the working examples thereof. However, a product

with a low kinematic viscosity is not obtained.

Patent Document 3 discloses a method for producing an α-olefin polymer, in which an α-olefin having 10 carbon atoms is polymerized with a metallocene catalyst, and a non-crosslinked metallocene catalyst is used in the working examples thereof. However, the product contains a dimer component in an amount of approximately 20%, and thus the amount of a VOC (volatile organic compound) component is disadvantageously large.

Patent Document 4 discloses a method for producing an α-olefin polymer, in which an α-olefin having 10 or more carbon atoms is polymerized with a metallocene catalyst, and a catalyst containing a meso double-crosslinked complex having two different crosslinking groups (Et/Et) is used in the working examples thereof. However, the amount of the trimer component is smaller than the dimer component, and thus the amount of a VOC component is disadvantageously large.

Patent Document 5 discloses a method for producing an α-olefin polymer, in which an α-olefin having 5 or more carbon atoms is polymerized with a metallocene catalyst, and a catalyst containing a non-crosslinked or crosslinked, substituted or unsubstituted catalyst is used in the working examples thereof. However, the polymer ends are terminated with a 2,1-bond (7% by mol of more of the 1,2-disubstituted component is contained), which is structurally different from the present invention.

The present invention has been developed under the circumstances, and an object thereof is to provide an α-olefin oligomer that contains a larger amount of a trimer component but a smaller amount of a hexamer while having a low viscosity, not in accordance with the Schulz-Flory distribution and a method for producing the same.

[Means for solving the Problems]

As a result of earnest investigations made by the inventors, it has been found that the above and other objects are achieved by using a particular double-crosslinked meso complex, and thus the present invention has been completed.

The present invention relates to an α-olefin oligomer and a method for producing an α-olefin oligomer described below.

1. An α-olefin oligomer having:

   (1) a trimer component proportion C3 (% by mass) that is larger than a theoretical value obtained from the S-F distribution,
   (2) a hexamer component proportion C6 (% by mass) that is smaller than a theoretical value obtained from the S-F distribution,
   (3) a kinematic viscosity at 100°C of 20 $mm^2/s$ or less,
   (4) a meso triad fraction [mm] measured by $^{13}C$-NMR of 40% by mol of less,
   (5) from 0.2 to 1.0 of a vinylidene group per one molecule,
   and
   (6) an average carbon number of a monomer unit of from 6 to 30,

   wherein the S-F distribution is calculated based on a chain propagation probability α calculated from a dimer component proportion C2 (% by mass).

2. (A) A meso transition metal compound represented by the following formula (I):

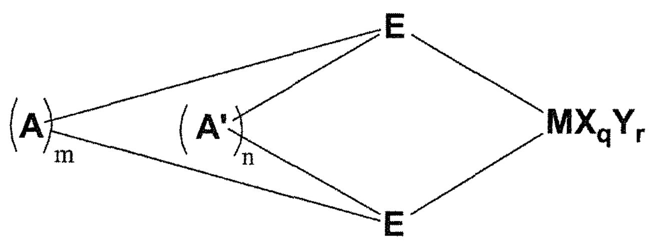

(I)

   wherein in the formula (I),

   M represents a metal element of Groups 3 to 10 in the Periodic Table;
   X represents a σ-bonding ligand, in which when there are plural ligands represented by X, the plural ligands may be the same as or different from each other;
   Y represents a Lewis base, in which when there are plural Lewis bases represented by Y, the plural Lewis bases may be the same as or different from each other;

A and A' each represent a crosslinking group selected from a hydrocarbon group having from 1 to 20 carbon atoms, a halogen-containing hydrocarbon group having from 1 to 20 carbon atoms, a silicon-containing group, a germanium-containing group and a tin-containing group, in which A and A' are different from each other;
m and n each represent an integer of 1 or more, in which m and n are different from each other, and m + n is 3 or more;
q represents an integer of from 1 to 5, which is ((valency of M) - 2);
r represents an integer of from 0 to 3; and
E represents a group represented by the following formula (II), in which two groups represented by E may be the same as or different from each other:

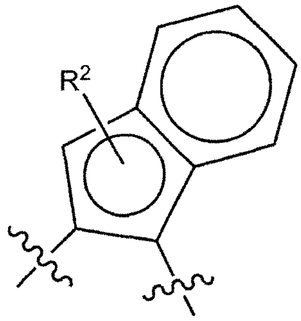

(II)

wherein in the formula (II),
$R^2$ each independently represent a group selected from a hydrogen atom, a halogen atom, a hydrocarbon group having from 1 to 20 carbon atoms, a halogen-containing hydrocarbon group having from 1 to 4 carbon atoms, a silicon-containing group and a hetero atom-containing group; and
the bonds shown with the wavy line represent bonds to $-(A)_m-$ and $-(A')_n-$.

3. The α-olefin oligomer according to the item 1, wherein the α-olefin oligomer is obtained with a polymerization catalyst containing:

(A) a meso transition metal compound represented by the following formula (I), and
(B) at least one component selected from (B-1) a compound that is capable of forming an ionic complex through reaction with the transition metal compound as the component (A) or a derivative thereof, and (B-2) aluminoxane:

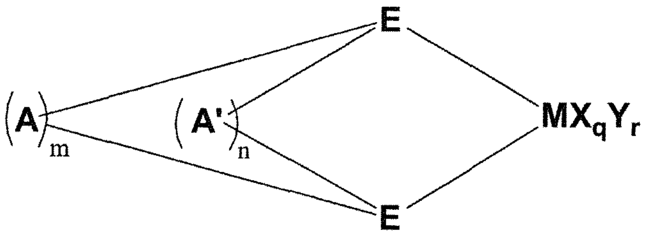

(I)

wherein in the formula (I),

M represents a metal element of Groups 3 to 10 in the Periodic Table;
X represents a σ-bonding ligand, in which when there are plural ligands represented by X, the plural ligands may be the same as or different from each other;
Y represents a Lewis base, in which when there are plural Lewis bases represented by Y, the plural Lewis bases may be the same as or different from each other;
A and A' each represent a crosslinking group selected from a hydrocarbon group having from 1 to 20 carbon atoms, a halogen-containing hydrocarbon group having from 1 to 20 carbon atoms, a silicon-containing group, a germanium-containing group and a tin-containing group, in which A and A' are different from each other;
m and n each represent an integer of 1 or more, in which m and n are different from each other, and m + n is 3 or more;
q represents an integer of from 1 to 5, which is ((valency of M) - 2);

r represents an integer of from 0 to 3; and
E represents a group represented by the following formula (II), in which two groups represented by E may be the same as or different from each other:

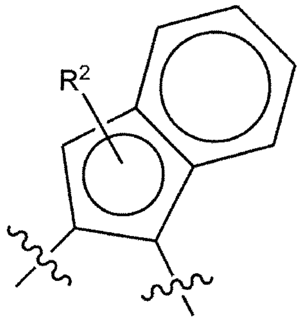

(II)

wherein in the formula (II),
$R^2$ each independently represent a group selected from a hydrogen atom, a halogen atom, a hydrocarbon group having from 1 to 20 carbon atoms, a halogen-containing hydrocarbon group having from 1 to 4 carbon atoms, a silicon-containing group and a hetero atom-containing group; and
the bonds shown with the wavy line represent bonds to $-(A)_m-$ and $-(A')_n-$.

4. A method for producing an $\alpha$-olefin oligomer, using a polymerization catalyst containing:

(A) a meso transition metal compound represented by the following formula (I), and
(B) at least one component selected from (B-1) a compound that is capable of forming an ionic complex through reaction with the transition metal compound as the component (A) or a derivative thereof, and (B-2) aluminoxane:

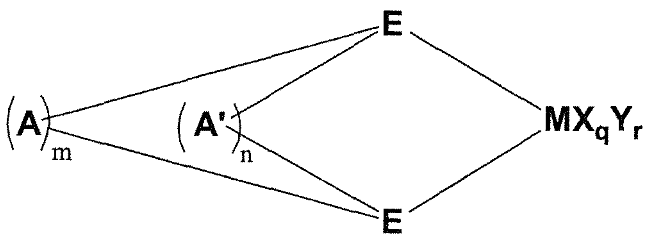

(I)

wherein in the formula (I),

M represents a metal element of Groups 3 to 10 in the Periodic Table;
X represents a $\sigma$-bonding ligand, in which when there are plural ligands represented by X, the plural ligands may be the same as or different from each other;
Y represents a Lewis base, in which when there are plural Lewis bases represented by Y, the plural Lewis bases may be the same as or different from each other;
A and A' each represent a crosslinking group selected from a hydrocarbon group having from 1 to 20 carbon atoms, a halogen-containing hydrocarbon group having from 1 to 20 carbon atoms, a silicon-containing group, a germanium-containing group and a tin-containing group, in which A and A' are different from each other;
m and n each represent an integer of 1 or more, in which m and n are different from each other, and m + n is 3 or more;
q represents an integer of from 1 to 5, which is ((valency of M) - 2);
r represents an integer of from 0 to 3; and
E represents a group represented by the following formula (II), in which two groups represented by E may be the same as or different from each other:

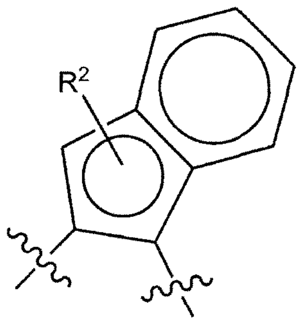

(II)

wherein in the formula (II),

$R^2$ each independently represent a group selected from a hydrogen atom, a halogen atom, a hydrocarbon group having from 1 to 20 carbon atoms, a halogen-containing hydrocarbon group having from 1 to 4 carbon atoms, a silicon-containing group and a hetero atom-containing group; and

the bonds shown with the wavy line represent bonds to $-(A)_m-$ and $-(A')_n-$.

5. The method for producing an α-olefin oligomer according to the item 4, wherein the polymerization catalyst is a catalyst obtained by making at least the component (A), the component (B) and (C) an organoaluminum compound into contact with each other in advance.

6. The method for producing an α-olefin oligomer according to the item 5, wherein the polymerization catalyst is a catalyst obtained by making at least the component (A), the component (B), the component (C) and (D) an α-olefin having from 3 to 18 carbon atoms into contact with each other in advance.

7. The method for producing an α-olefin oligomer according to any one of the items 4 to 6, wherein in the formula (I), the crosslinking group represented by $-(A)_m-$ is represented by the following formula (IV), and the crosslinking group represented by $-(A')_n-$ is represented by the following formula (IV'):

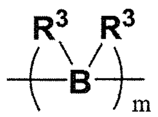 (A) (IV) 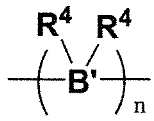 (A') (IV')

wherein in the formulae (IV) and (IV'),

B and B' each independently represent a carbon atom, a silicon atom, a germanium atom or a tin atom;

$R^3$ and $R^4$ each independently represent a hydrogen atom, an aliphatic hydrocarbon group having from 1 to 20 carbon atoms, an aromatic hydrocarbon group having from 6 to 20 carbon atoms, an oxygen-containing group having from 1 to 20 carbon atoms, an amine-containing group having from 1 to 20 carbon atoms or a halogen-containing group having from 1 to 20 carbon atoms; and

m and n each independently represent an integer of 1 or more.

8. The method for producing an α-olefin oligomer according to any one of the items 4 to 7, wherein the aluminoxane as the component (B-2) is a linear aluminoxane represented by the following formula (VII) and/or a cyclic aluminoxane represented by the following formula (VIII):

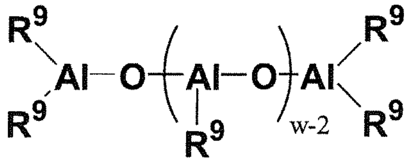

(VII)

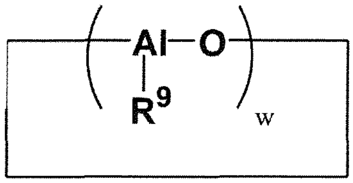

(VIII)

wherein $R^9$ represents a hydrocarbon group having from 1 to 20 carbon atoms or a halogen atom, in which groups represented by $R^9$ may be the same or different; and w represents a number of from 2 to 50, which is an average polymerization degree.

9. The method for producing an α-olefin oligomer according to any one of the items 4 to 8, wherein reaction is performed at a temperature of from 40 to 200°C.

10. The method for producing an α-olefin oligomer according to any one of the items 4 to 9, wherein reaction is performed under a hydrogen pressure of from the atmospheric pressure to 10 MPa (G).

[Advantages of the Invention]

According to the present invention, an α-olefin oligomer and a method for producing the same are provided, in which the α-olefin oligomer has low regularity and contains a larger amount of a trimer component but a smaller amount of a hexamer while having a low viscosity, not in accordance with the Schulz-Flory distribution.

[Embodiments for carrying out the Invention]

α-Olefin Oligomer

The α-olefin oligomer according to the present invention satisfies the conditions (1) to (6) described above, and may be obtained by the method for producing an α-olefin oligomer according to the present invention.

The α-olefin oligomer of the present invention will be described in detail below including the conditions (1) to (6).

(1) Trimer Component Proportion C3

The α-olefin oligomer of the present invention has a trimer component proportion C3 (unit: % by mass) that is larger than a theoretical value obtained from the Schulz-Flory distribution (which may be hereinafter abbreviated as the S-F distribution).

In an ordinary α-olefin polymer in accordance with the Schulz-Flory distribution, the theoretical values of the amounts of the oligomer components (such as a dimer, a trimer, a tetramer and the like) with respect to the total amount of the oligomer can be calculated from the chain propagation probability α. Specifically, where the probability that an n-mer (wherein n is an integer of 2 or more) becomes an n+1-mer in polymerization reaction is designated as the chain propagation probability α, the probability that an n-mer does not become an n+1-mer but undergoes chain transfer (polymerization termination) is expressed by (1-α), and thus the molar ratio of the n-mer component in the α-olefin oligomer composition is obtained from the following expression (1) based on the chain propagation probability α.

$$\text{(molar ratio of n-mer component)} = \alpha^{(n-2)} \times (1-\alpha) \qquad (1)$$

wherein n represents an integer of 2 or more.

The chain propagation probability α is defined as a value calculated from the dimer component proportion C2 (unit: % by mass).

Where the molecular weight of the monomer unit of the α-olefin oligomer is represented by m, the mass values of the n-mer components are expressed by the following expression (i).

$$\text{(mass of n-mer component)} = n \times m \times \alpha^{(n-2)} \times (1-\alpha) \qquad (i)$$

Similarly, the sum of the n-mer components (i.e., the mass of the α-olefin oligomer) is expressed by the following expression (ii).

$$m(1-\alpha)\sum_{n=2}^{\infty} n\,\alpha^{(n-2)} \quad \text{(ii)}$$

Accordingly, the n-mer component proportions Cn (unit: % by mass) is expressed by the following expression (I) with the formula (i) as the numerator and the formula (ii) as the denominator.

$$\frac{n\,\alpha^{(n-2)}}{\sum_{n=2}^{\infty} n\,\alpha^{(n-2)}} \quad \text{(I)}$$

The chain propagation probability $\alpha$ is calculated based on the measured value of the dimer component proportion C2 of the $\alpha$-olefin oligomer according to the expression (I), and the theoretical values of the trimer component proportion C3 and the hexamer component proportion C6 described later are calculated based on the resulting chain propagation probability $\alpha$ and the target n value according to the expression (I), and are compared to the measured values thereof of the $\alpha$-olefin oligomer.

The $\alpha$-olefin oligomer of the present invention has a trimer component proportion C3 that is larger than the theoretical value obtained from the Schulz-Flory distribution.

The $\alpha$-olefin oligomer of the present invention preferably has a trimer component proportion C3 that is larger than the theoretical value obtained from the S-F distribution by 5% or more, and more preferably by 10% or more.

When the trimer component proportion C3 does not satisfy the condition, the dimer component proportion C2 is too large as compared to the trimer component proportion C3, whereby an $\alpha$-olefin oligomer that is suitable for the target application may not be obtained even through the $\alpha$-olefin oligomer is subjected to purification after production, and the deterioration in productivity and the increase of the amount of waste materials may cause environmental deterioration. Furthermore, for example, in the case where an $\alpha$-olefin oligomer of an $\alpha$-olefin having 16 or more carbon atoms contains a large amount of a dimer component, the dimer component is a component having 32 or more carbon atoms and thus is difficult to be removed through such a measure as distillation or the like, but remains in the final product. Accordingly, the dimer component remaining in a large amount causes melting point depression and broadens the melting point distribution, which disadvantageously results in a large amount of sticky components.

(2) Hexamer Component Proportion C6

The $\alpha$-olefin oligomer of the present invention has a hexamer component proportion C6 (unit: % by mass) that is smaller than a theoretical value obtained from the S-F distribution.

The n-mer component proportion Cn can be obtained, for example, by gas chromatograph (GC).

(3) Kinematic viscosity at 100°C

The $\alpha$-olefin oligomer of the present invention has a kinematic viscosity at 100°C measured according to JIS K2283 of 20 $mm^2/s$ or less, preferably in a range of from 2 to 20 $mm^2/s$, more preferably in a range of from 3 to 15 $mm^2/s$, and further preferably in a range of from 4 to 10 $mm^2/s$.

When the kinematic viscosity at 100° is 2 $mm^2/s$ or more, the amount of VOC (volatile organic compound) may be small, and when it is 20 $mm^2/s$ or less, the dynamic loss due to viscous resistance upon using as a lubricating oil may be suppressed, which may improve the fuel consumption.

(4) Meso Triad Fraction [mm]

The $\alpha$-olefin oligomer of the present invention has a meso triad fraction [mm] measured by $^{13}C$-NMR of 40% by mol of less, preferably from 25 to 40% by mol, more preferably from 30 to 40% by mol, and further preferably from 32 to 38% by mol. When the meso triad fraction [mm] exceeds 40% by mol, the $\alpha$-olefin oligomer may be inferior in low temperature characteristics. It is more preferred from the standpoint of the low temperature characteristics that the meso triad fraction [mm] is 25% by mol or more and less than 40% by mol.

(5) Amount of Vinylidene Group

The α-olefin oligomer of the present invention has from 0.2 to 1.0 of a vinylidene group per one molecule, preferably from 0.3 to 1.0 of a vinylidene group per one molecule, and more preferably from 0.5 to 1.0 of a vinylidene group per one molecule. When the amount of a vinylidene group is in the range, the α-olefin oligomer may be subjected to a secondary modification or the like for imparting polarity, and may be useful particularly as a wax component. For achieving the aforementioned number of a vinylidene group, the reaction conditions including the temperature, the amount of hydrogen, and the like may be controlled.

(6) Average Carbon Number of Monomer Unit

The α-olefin oligomer of the present invention has an average carbon number of a monomer unit of from 6 to 30, preferably from 6 to 24, and more preferably from 6 to 18. The α-olefin oligomer of the present invention has a low polymerization degree, and when the average carbon number is less than 6, the α-olefin oligomer may be difficult to be a liquid polymer, and the amount of VOC (volatile organic compound) may be increased, which makes application to a lubricating oil difficult. When the average carbon number exceeds 30, the monomer may be in a solid state, and the low temperature characteristics may be deteriorated.

In the case where two or more kinds of α-olefins are used as a raw material, the average carbon number herein means the molar average carbon number of the monomer units, and in the case where one kind of an α-olefin is used as a raw material, the average carbon number means the carbon number of the α-olefin used. For example, a copolymer obtained from 1-hexene and 1-dodecene at a molar ratio of 1/2 has an average carbon number of 10.

In the α-olefin oligomer of the present invention, the proportion of an α-olefin unit having 4 or more carbon atoms is preferably 100% by mol. Examples of a monomer unit other than the α-olefin unit having 4 or more carbon atoms include an α-olefin unit having 3 or less carbon atoms. The α-olefin unit having 4 or more carbon atoms is preferably an α-olefin having from 6 to 16 carbon atoms, and more preferably an α-olefin having from 6 to 14 carbon atoms, for the application to a lubricating oil. The α-olefin may be used solely, or plural kinds thereof may be copolymerized. In the case where an α-olefin having 16 or more carbon atoms is used, an α-olefin having from 4 to 12 carbon atoms may be copolymerized therewith, thereby making the average carbon number in a range of from 6 to 14. Specific examples of the α-olefin having 6 or more carbon atoms include 1-hexene, 1-octene, 1-decene, 1-undecene, 1-dodecene, 1-tridecene, 1-tetradecene, 1-pentadecene, 1-hexadecene, 1-heptadecene, 1-octadecene, 1-nonadecene, 1-eicosene, 1-tetracosene, 1-hexacosene, 1-octacosene and 1-triacontene, which may be used solely or as a combination of two or more kinds thereof. In the present invention, these raw material monomers may be used, thereby providing an α-olefin oligomer that is useful as a lubricating oil component. The α-olefins may be branched olefins.

The α-olefin oligomer of the present invention generally has a weight average molecular weight (Mw) of 9,000 or less, preferably from 100 to 9,000 more preferably from 300 to 7, 000, and particularly preferably from 500 to 5,000. When the weight average molecular weight (Mw) is 9,000 or less, the viscosity thereof may not be too large, and the α-olefin oligomer may exhibit characteristics as a low molecular weight material.

The α-olefin oligomer of the present invention generally has a molecular weight distribution (Mw/Mn) of 1.5 or less, preferably from 1.0 to 1.5, and more preferably from 1.1 to 1.5. When the molecular weight distribution (Mw/Mn) is 1.5 or less, a narrow compositional distribution may be obtained, and the content of the compound having the target property may be increased.

The weight average molecular weight (Mw) and the molecular weight distribution (Mw/Mn) may be obtained by the GPC method (polystyrene conversion).

The α-olefin oligomer of the present invention preferably has a viscosity index (VI) of 150 or more, and more preferably 170 or more. When the viscosity index (VI) is less than 150, the cold startability may be deteriorated. A higher viscosity index may provide a lower temperature dependency and thus is preferred as a lubricating oil.

The α-olefin oligomer of the present invention preferably has a proportion of a dimer component of 60% by mass or less, and more preferably 30% by mass or less. When the proportion of a dimer component exceeds 60% by mass, the dimer component formed in a large amount may cause such problems that an α-olefin oligomer that is suitable for the target application may not be obtained even through the α-olefin oligomer is subjected to purification after production, and the deterioration in productivity and the increase of the amount of waste materials may cause environmental deterioration. When the aforementioned condition is satisfied, the α-olefin oligomer of the present invention has a reduced dimer component proportion and a small molecular weight distribution value close to the homogeneous composition, thereby providing a product having a target viscosity range, and furthermore is useful as a wax component and a lubricating oil component owing to the substantial absence of a component that deteriorates the capabilities.

The proportion of the dimer component may be obtained, for example, by gas chromatograph (GC).

The α-olefin oligomer of the present invention is useful mainly as a wax component and a lubricating oil component, and is useful particularly as a releasing agent and an ink component for a toner, a modifier for a resin, a pressure

sensitive adhesive component, an adhesive component, a lubricating oil component, an organic-inorganic composite material, a heat storage material, a modifier for a fuel oil, such as light oil, a modifier for asphalt, and high performance wax. In addition to the above, the α-olefin oligomer is also useful as components of cosmetics (such as lip stick, pomade, creme, eyebrowpencil, eye shadow, hair oil, facial mask, shampoo, and conditioner), a medical material (such as ointment, suppository, emulsion, surgical bandage material, and wet dressing material), a stationery material (such as crayon, crayon pastel, pencil, and carbon paper), a glazing material (for wood, furniture, leather, automobile, paper, confectionery, fibers and the like), a candle, a leather cream, a textile oil, a confectionery material, a model material, a sculpture material, a leather finishing material, an insulating wax paper material, a musical instrument, a printing material for grafting wax, a material for producing a casting mold, a wax coating material for fruits, grease, ski wax, a batik dyeing material, a polishing material, car wax, a metalworking fluid, a rubber antioxidant, a tire, an adhesive, processed paper, a heat storage material, agrichemicals, fertilizer, a polishing material (for metal and stainless steel), an oily lubricant (such as a grease, a releasing agent and a paint), dental wax, a fixing material (for a lens and for embedment), and the like.

Method for producing α-Olefin Oligomer

The method for producing an α-olefin oligomer of the present invention uses a polymerization catalyst containing:

(A) a meso transition metal compound represented by the following formula (I), and
(B) at least one component selected from (B-1) a compound that is capable of forming an ionic complex through reaction with the transition metal compound as the component (A) or a derivative thereof, and (B-2) aluminoxane:

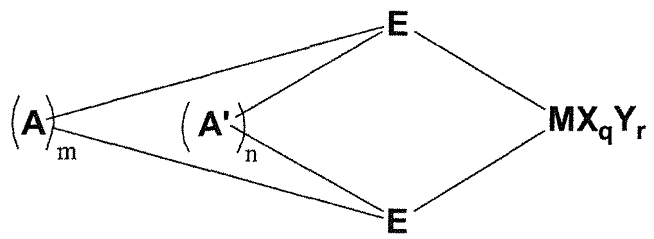

(I)

wherein in the formula (I),

M represents a metal element of Groups 3 to 10 in the Periodic Table;
X represents a σ-bonding ligand, in which when there are plural ligands represented by X, the plural ligands may be the same as or different from each other;
Y represents a Lewis base, in which when there are plural Lewis bases represented by Y, the plural Lewis bases may be the same as or different from each other;
A and A' each represent a crosslinking group selected from a hydrocarbon group having from 1 to 20 carbon atoms, a halogen-containing hydrocarbon group having from 1 to 20 carbon atoms, a silicon-containing group, a germanium-containing group and a tin-containing group, in which A and A' are different from each other;
m and n each represent an integer of 1 or more, in which m and n are different from each other, and m + n is 3 or more;
q represents an integer of from 1 to 5, which is ((valency of M) - 2);
r represents an integer of from 0 to 3; and
E represents a group represented by the following formula (II), in which two groups represented by E may be the same as or different from each other.

It is preferred that one of m and n is 1, and the other is an integer of 2 or more, and it is more preferred that one of m and n is 1, and the other is 2.

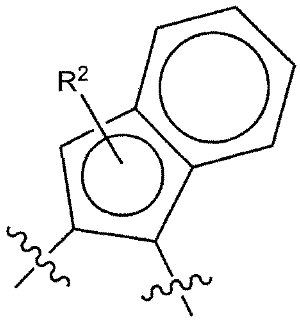

(II)

wherein in the formula (II),

$R^2$ each independently represent a group selected from a hydrogen atom, a halogen atom, a hydrocarbon group having from 1 to 20 carbon atoms, a halogen-containing hydrocarbon group having from 1 to 4 carbon atoms, a silicon-containing group and a hetero atom-containing group; and
the bonds shown with the wavy line represent bonds to- $(A)_m$- and -$(A')_n$-.

In the formula (I), specific examples of the metal element of Groups 3 to 10 in the Periodic Table represented by M include titanium, zirconium, hafnium, yttrium, vanadium, chromium, manganese, nickel, cobalt, palladium and a lanthanoid metal. Among these, titanium, zirconium and hafnium are preferred from the standpoint of the olefin polymerization activity, and zirconium is most preferred from the standpoint of the yield of the terminal vinylidene group and the catalytic activity.

X represents a σ-bonding ligand, in which when there are plural ligands represented by X, the plural ligands may be the same as or different from each other, and may be crosslinked to the other X, E or Y. Specific examples of X include a halogen atom, a hydrocarbon group having from 1 to 20 carbon atoms, an alkoxy group having from 1 to 20 carbon atoms, an aryloxy group having from 6 to 2 0 carbon atoms, an amide group having from 1 to 20 carbon atoms, a silicon-containing group having from 1 to 20 carbon atoms, a phosphide group having from 1 to 20 carbon atoms, a sulfide group having from 1 to 20 carbon atoms and an acyl group having from 1 to 20 carbon atoms.

Examples of the halogen atom include a chlorine atom, a fluorine atom, a bromine atom and an iodine atom. Specific examples of the hydrocarbon group having from 1 to 20 carbon atoms include an alkyl group, such as a methyl group, an ethyl group, a propyl group, a butyl group, a hexyl group, a cyclohexyl group and an octyl group; an alkenyl group, such as vinyl group, a propenyl group and a cyclohexenyl group; an arylalkyl group, such as a benzyl group, a phenylethyl group and a phenylpropyl group; and an aryl group, such as a phenyl group, a tolyl group, a dimethylphenyl group, a trimethylphenyl group, an ethylphenyl group, a propylphenyl group, a biphenyl group, a naphthyl group, a methylnaphthyl group, an anthracenyl group and a phenanthrenyl group. Among these, an alkyl group, such as a methyl group, an ethyl group and a propyl group, and an aryl group, such as a phenyl group, are preferred.

Examples of the alkoxy group having from 1 to 20 carbon atoms include an alkoxy group, such as a methoxy group, an ethoxy group, a propoxy group and a butoxy group. Examples of the aryloxy group having from 6 to 20 carbon atoms include a phenoxy group, a methylphenoxy group and a dimethylphenoxy group.

Examples of the amide group having from 1 to 20 carbon atoms include an alkylamide group, such as a dimethylamide group, a diethylamide group, a dipropylamide group, a dibutylamide group, a dicyclohexylamide group and a methylethylamide group and an alkenyl amide group, such as a divinyl amide group, a dipropenyl amide group and dicyclohexenyl amide group; an arylalkylamide group, such as a dibenzylamide group, a phenylethylamide group and a phenylpropylamide group; and an arylamide group, such as a diphenylamide group and a dinaphthylamide group.

Examples of the silicon-containing group having from 1 to 20 carbon atoms include a mono-hydrocarbon-substituted silyl group, such as a methylsilyl group and a phenylsilyl group; a di-hydrocarbon-substituted silyl group, such as dimethylsilyl group and a diphenylsilyl group; a tri-hydrocarbon-substituted silyl group, such as a trimethylsilyl group, a triethylsilyl group, a tripropylsilyl group, a tricyclohexylsilyl group, a triphenylsilyl group, a dimethylphenylsilyl group, a methyldiphenylsilyl group, a tritolylsilyl group and a trinaphthylsilyl group; a hydrocarbon-substituted silyl ether group, such as a trimethylsilyl ether group; a silicon-substituted alkyl group, such as a trimethylsilylmethyl group; and a silicon-substituted aryl group, such as a trimethylsilylphenyl group. Among these, a trimethylsilylmethyl group, a phenyldimethylsilylethyl group and the like are preferred.

Examples of the phosphide group having from 1 to 20 carbon atoms include an alkylphosphide group, such as a dimethylphosphide group, a diethylphosphide group, a dipropylphosphide group, a dibutylphosphide group, a dicyclohexylphosphide group and a dioctylphosphide group; an alkenylphosphide group, such as divinylphosphide group, a dipropenylphosphide group and a dicyclohexenylphosphide group; an arylalkylphosphide group, such as a dibenzylphosphide group and a bis(phenylethylphenyl)phosphide group; and an arylphosphide group, such as a diphenylphosphide group, a ditolylphosphide group and a dinaphthylphosphide group.

Examples of the sulfide group having from 1 to 20 carbon atoms include an alkylsulfide group, such as a methylsulfide group, an ethylsulfide group, a propylsulfide group, a butylsulfide group, a hexylsulfide group, a cyclohexylsulfide group and an octylsulfide group; an alkenylsulfide group, such as a vinylsulfide group, a propenylsulfide group and a cyclohexenylsulfide group; an arylalkylsulfide group, such as a benzylsulfide group, a phenylethylsulfide group and a phenylpropylsulfide group; and an arylsulfide group, such as a phenylsulfide group, a tolylsulfide group, a dimethylphenylsulfide group, a trimethylphenylsulfide group, an ethylphenylsulfide group, a propylphenylsulfide group, a biphenylsulfide group, a naphthylsulfide group, a methylnaphthylsulfide group, an anthracenylsulfide group and a phenanthrylsulfide group.

Examples of the acyl group having from 1 to 20 carbon atoms include an alkylacyl group, such as a formyl group, an acetyl group, a propionyl group, a butylyl group, a valeryl group, a palmitoyl group, a stearoyl group and an oleoyl group; an arylacyl group, such as a benzoyl group, a toluoyl group, a salicyloyl group, a cinnamoyl group, a naphthoyl group and a phthaloyl group; an oxalyl group, a malonyl group, a succinyl group and the like, which are derived from oxalic acid, malonic acid, a succinic acid and the like.

Y represents a Lewis base, in which when there are plural Lewis bases represented by Y, the plural Lewis bases may be the same as or different from each other, and may be crosslinked to the other Y, E or X. Specific examples of the Lewis base represented by Y include an amine compound, an ether compound, a phosphine compound and a thioether compound.

Examples of the amine include an amine having from 1 to 20 carbon atoms, and specific examples thereof include an alkylamine, such as methylamine, ethylamine, propylamine, butylamine, cyclohexyl amine, methylethylamine, dimethylamine, diethylamine, dipropylamine, dibutylamine and dicyclohexylamine; an alkenylamine, such as vinylamine, propenylamine, cyclohexenylamine, divinylamine, dipropenylamine and dicyclohexenylamine; an arylalkylamine, such as phenylethylamine and phenylpropylamine; and an arylamine, such as phenylamine, diphenylamine and dinaphthylamine.

Examples of the ether compound include an aliphatic single ether, such as methyl ether, ethyl ether, propyl ether, isopropyl ether, butyl ether, isobutyl ether, n-amyl ether and isoamyl ether; an aliphatic mixed ether, such as methyl ethyl ether, methyl propyl ether, methyl isopropyl ether, methyl n-amyl ether, methyl isoamyl ether, ethyl propyl ether, ethyl isopropyl ether, ethyl butyl ether, ethyl isobutyl ether, ethyl n-amyl ether and ethyl isoamyl ether; an aliphatic unsaturated ether compound, such as vinyl ether, allyl ether, methyl vinyl ether, methyl allyl ether, ethyl vinyl ether and ethyl allyl ether; an aromatic ether compound, such as anisole, phenetol, phenyl ether, benzyl ether, phenyl benzyl ether, $\alpha$-naphthyl ether and $\beta$-naphthyl ether; and a cyclic ether compound, such as ethylene oxide, propylene oxide, trimethylene oxide, tetrahydrofuran, tetrahydropyran and dioxane.

Examples of the phosphine compound include a phosphine having from 1 to 20 carbon atoms, and specific examples thereof include an alkylphosphine, for example, a mono-hydrocarbon-substituted phosphine, such as methylphosphine, ethylphosphine, propylphosphine, butylphosphine, hexylphosphine, cyclohexylphosphine and octylphosphine; a di-hydrocarbon-substituted phosphine, such as dimethylphosphine, diethylphosphine, dipropylphosphine, dibutylphosphine, dihexylphosphine, dicyclohexylphosphine and dioctylphosphine; and a tri-hydrocarbon-substituted phosphine, such as trimethylphosphine, triethylphosphine, tripropylphosphine, tributylphosphine, trihexylphosphine, tricyclohexylphosphine and trioctylphosphine; a monoalkenylphosphine, such as vinylphosphine, propenylphosphine and cyclohexenylphosphine; a dialkenylphosphine formed by substituting hydrogen atoms of a phosphine with two alkenyl groups; a trialkenylphosphine formed by substituting hydrogen atoms of a phosphine with three alkenyl groups; an arylalkylphosphine, such as benzylphosphine, phenylethylphosphine and phenylpropylphosphine; a diarylalkylphosphine or an aryldialkylphosphine formed by substituting hydrogen atoms of a phosphine with three aryl or alkenyl groups; and an arylphosphine, for example, phenylphosphine, tolylphosphine, dimethylphenylphosphine, trimethylphenylphosphine, ethylphenylphosphine, propylphenylphosphine, biphenylphosphine, naphthylphosphine, methylnaphthylphosphine, anthracenylphosphine and phenanthrylphosphine; a di(alkylaryl)phosphine formed by substituting hydrogen atoms of a phosphine with two alkylaryl groups; and a tri(alkylaryl)phosphine formed by substituting hydrogen atoms of a phosphine with three alkylaryl groups. Examples of the thioether compound include the sulfide compounds described above.

In the formula (I), the crosslinking group represented by $-(A)_m-$ is preferably represented by the following formula (IV), and the crosslinking group represented by $-(A')_n-$ is preferably represented by the following formula (IV'):

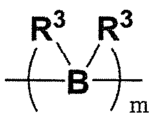 (A) (IV) 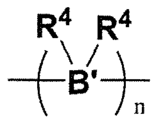 (A') (IV')

wherein in the formulae (IV) and (IV'),

B and B' each independently represent a carbon atom, a silicon atom, a germanium atom or a tin atom;
$R^3$ and $R^4$ each independently represent a hydrogen atom, an aliphatic hydrocarbon group having from 1 to 20 carbon atoms, an aromatic hydrocarbon group having from 6 to 20 carbon atoms, an oxygen-containing group having from 1 to 20 carbon atoms, an amine-containing group having from 1 to 20 carbon atoms or a halogen-containing group having from 1 to 20 carbon atoms; and
m and n each independently represent an integer of 1 or more.

Specific examples of the crosslinking groups represented by $-(A)_m-$ and $-(A')_n-$ include an ethylene group, a tetramethylethylene group, a 1,2-cyclohexylene group, a tetramethyldisilylene group, a dimethylsilylenemethylene group, dimethylsilyleneisopropylidene group and a tetramethyldigermylene group. Among these, an ethylene group and a dimethylsilylene group are preferred since the polymerization activity is further enhanced, and more specifically, it is preferred that one of the crosslinking groups represented by $-(A)_m-$ and $-(A')_n-$ is an ethylene group, and the other thereof is a dimethylsilylene group.

In the meso transition metal compound represented by the formula (I), a transition metal compound having a double-crosslinked bisindenyl derivative as a ligand represented by the following formula (III) is preferred.

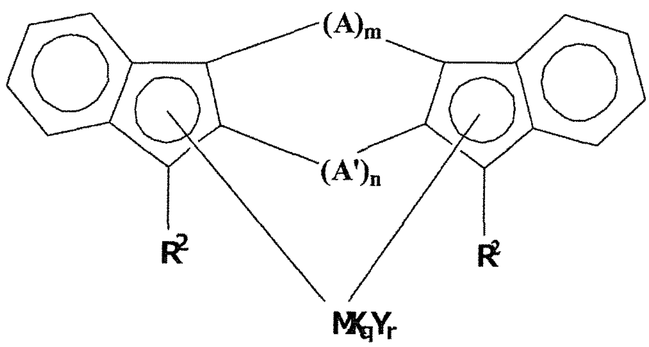

· · · (III)

The complex structure in the basic skeleton shown above is a meso type having 1,1'- and 2,2'-crosslinked structure.

In the formula (III), M, $-(A)_m-$, $-(A')_n-$, $R^2$, q and r have the same meanings as in the formula (I).

Specific examples of the transition metal compound represented by the formula (I) include (1,1'-ethylene) (2,2'-tetramethyldisilylene) bis(indenyl)zirconium dichloride, (1,1'-tetramethyldisilylene) (2,2'-ethylene) bis(indenyl)zirconium dichloride, (1,1'-tetramethylethylene) (2,2'-tetramethyldisilylene) bis(indenyl)zirconium dichloride, (1,1'-tetramethyldisilylene) (2,2'-tetramethylethylene) bis(indenyl)zirconium dichloride, (1,1'-dimethylsilylenemethylene) (2,2'-ethylene) bis(3-methylindenyl)zirconium dichloride, (1,1'-ethylene) (2,2'-dimethylsilylenemethylene) bis(3-methylindenyl)zirconium dichloride, (1,1'-ethylene) (2,2'-tetramethyldigermylene) bis(indenyl)zirconium dichloride, (1,1'-tetramethyldigermylene) (2,2'-ethylene) bis(indenyl)zirconium dichloride, (1,1'-cyclohexylene) (2,2'-tetramethyldisilylene) bis(indenyl)zirconium dichloride, (1,1'-tetramethyldisilylene) (2,2'-cyclohexylene) bis(indenyl)zirconium dichloride,

(1,1'-ethylene) (2,2'-tetramethyldisilylene) bis(3-methylindenyl)zirconium dichloride, (1,1'-tetramethyldisilylene) (2,2'-ethylene) bis(3-methylindenyl)zirconium dichloride, (1,1'-tetramethylethylene) (2,2'-tetramethyldisilylene) bis(3-methylindenyl)zirconium dichloride, (1,1'-tetramethyldisilylene) (2,2'-tetramethylethylene) bis(3-methylindenyl)zirconium dichloride, (1,1'-dimethylsilylenemethylene) (2,2'-ethylene) bis(3-methylindenyl)zirconium dichloride, (1,1'-ethylene) (2,2'-dimethylsilylenemethylene) bis(3-methylindenyl)zirconium dichloride, (1,1'-ethylene) (2,2'-tetramethyldigermylene) bis(3-methylindenyl)zirconium dichloride, (1,1'-tetramethyldigermylene) (2,2'-ethylene) bis (3-methylindenyl) zirconium chloride, and compounds formed by substituting zirconium of the aforementioned compounds with titanium or hafnium, but the transition metal compound in the present invention is not limited to these compounds. Examples of the transition metal compound also include similar compounds as above that have a metal element of the other Groups or a lanthanoid metal.

The component (B-1) in the component (B) may be any compound that is capable of forming an ionic complex through reaction with the meso transition metal compound as the component (A) or a derivative thereof, and compounds represented by the following formulae (V) and (VI) are preferably used:

$$((L^1\text{-}R^4)^{k+})_a((Z)^-)_b \qquad (V)$$

$$((L^2)^{k+})_a((Z)^-)_b \qquad (VI)$$

wherein $L^2$ is $M^2$, $R^5R^6M^3$, $R^7{}_3C$ or $R^8M^3$.

In the formulae (V) and (VI),

$L^1$ represents a Lewis base;

$(Z)^-$ represents non-coordinating anions $(Z^1)^-$ and $(Z^2)^-$, wherein $(Z^1)^-$ represents an anion formed by bonding plural groups to an element, i.e., $(M^1G^1G^2...G^f)^-$ (wherein $M^1$ represents an element of Groups 5 to 15 in the Periodic Table, and preferably an element of Groups 13 to 15 in the Periodic Table; $G^1$ to $G^f$ each represent a hydrogen atom, a halogen atom, an alkyl group having from 1 to 20 carbon atoms, a dialkylamino group having from 2 to 4 0 carbon atoms, an alkoxy group having from 1 to 20 carbon atoms, an aryl group having from 6 to 20 carbon atoms, an aryloxy group having from 6 to 20 carbon atoms, an alkylaryl group having from 7 to 40 carbon atoms, an arylalkyl group having from 7 to 40 carbon atoms, a halogen-substituted hydrocarbon group having from 1 to 20 carbon atoms, an acyloxy group having from 1 to 20 carbon atoms, an organic metalloid group or a hetero atom-containing hydrocarbon group having from 2 to 20 carbon atoms, in which two or more of $G^1$ to $G^f$ may form a ring; and f represents an integer, which is ((valency of center metal $M^1$) + 1)), and $(Z^2)^-$ represents a conjugate base of a Bronsted acid having logarithm of the reciprocal of the acid dissociation constant (pKa) of -10 or less solely or a combination of a Bronsted acid and a Lewis acid, or a conjugate salt of an acid that is generally defined as a super acid, to which a Lewis base may be coordinated;

$R^4$ represents a hydrogen atom, an alkyl group having from 1 to 20 carbon atoms, an aryl group having from 6 to 20 carbon atoms, an alkylaryl group or an arylalkyl group;

$R^5$ and $R^6$ each represent a cyclopentadienyl group, a substituted cyclopentadienyl group, an indenyl group or a fluorenyl group;

$R^7$ represents an alkyl group having from 1 to 20 carbon atoms, an aryl group, an alkylaryl group or an arylalkyl group;

$R^8$ represents a macrocyclic ligand, such as tetraphenylporphyrin and phthalocyanine;

k represents an ion charge of $(L^1\text{-}R^4)$ and $(L^2)$, which is an integer of from 1 to 3;

a represents an integer of 1 or more;

b represents (k x a);

$M^2$ represents an element of Groups 1 to 3, 11 to 13 and 17 in the Periodic Table; and

$M^3$ represents an element of Groups 7 to 12 in the Periodic Table.

Specific examples of $L^1$ include an amine compound, such as ammonia, methylamine, aniline, dimethylamine, diethylamine, N-methylaniline, diphenylamine, N,N-dimethylaniline, trimethylamine, triethylamine, tri-n-butylamine, methyldiphenylamine, pyridine, p-bromo-N,N-dimethylaniline and p-nitro-N,N-dimethylaniline, a phosphine compound, such as triethylphosphine, triphenylphosphine and diphenylphosphine, a thioether compound, such as tetrahydrothiophene, an ester compound, such as ethyl benzoate, and a nitrile compound, such as acetonitrile and benzonitrile.

Specific examples of $R^4$ include a hydrogen atom, a methyl group, an ethyl group, a benzyl group and a trityl group, and specific examples of $R^5$ and $R^6$ include a cyclopentadienyl group, a methylcyclopentadienyl group, an ethylcyclopentadienyl group and a pentamethylcyclopentadienyl group. Specific examples of $R^7$ include a phenyl group, a p-tolyl group and a p-methoxyphenyl group, and specific examples of $R^8$ include tetraphenylporphyrin, phthalocyanine, allyl and methallyl. Specific examples of $M^2$ include Li, Na, K, Ag, Cu, Br, I and $I_3$, and specific examples of $M^3$ include Mn, Fe, Co, Ni and Zn. Specif ic examples of $M^1$ in $(Z^1)^-$, i.e., $(M^1G^1G^2... G^f)^-$, include B, Al, Si, P, As and Sb, and preferably B and Al. Specific examples of $G^1$ and $G^2$ to $G^f$ include a dialkylamino group, such as dimethylamino group and a diethylamino group, an alkoxy group and an aryloxy group, such as a methoxy group, an ethoxy group, a n-butoxy group and a phenoxy group, a hydrocarbon group, such as a methyl group, an ethyl group, a n-propyl group, an isopropyl group, a n-butyl group, an isobutyl group, a n-octyl group, a n-eicosyl group, a phenyl group, a p-tolyl group, a benzyl group, a 4-t-butylphenyl group and a 3,5-dimethylphenyl group, a halogen atom, such as fluorine, chlorine, bromine and iodine, a hetero atom-containing hydrocarbon group, such as a p-fluorophenyl group, a 3,5-difluorophenyl group, a pentachlorophenyl group, a 3,4,5-trifluorophenyl group, a pentafluorophenyl group, a 3,5-bis(trifluoromethyl)phenyl group and a bis(trimethylsilyl)methyl group, an organic metalloid group, such as a pentamethylantimony group, a trimethylsilyl group, a trimethylgermyl group, a diphenylarsine group, a dicyclohexylantimony group and a diphenylboron group.

Specific examples of the non-coordinating anion, i.e., the conjugate base $(Z^2)^-$ of a Bronsted acid having pKa of -10 or less solely or a combination of the Bronsted acid and a Lewis acid, include trifluoromethanesulfonic acid anion $(CF_3SO_3)^-$, bis(trifluoromethanesulfonyl)methyl anion, bis(trifluoromethanesulfonyl)benzyl anion, bis(trifluoromethanesulfonyl)aminde, perchloric acid anion $(ClO_4)^-$, trifluoroacetic acid anion $(CF_3CO_2)^-$, hexafluoroantimony anion $(SbF_6)^-$, fluorosulfonic acid anion $(FSO_3)^-$, chlorosulfonic acid anion $(ClSO_3)^-$, fluorosulonic acid anion/pentafluoroantimony $(FSO_3/SbF_5)^-$, fluorosulfonic acid anion/pentafluoro arsenic $(FSO_3/AsF_5)^-$ and trifluoromethanesulfonic acid/pentafluoroantimony $(CF_3SO_3/SbF_5)^-$.

Specific examples of the ionic compound capable of forming an ionic complex through reaction with the transition metal compound as the component (A) or a derivative thereof, i.e., the compound of the component (B-1), include triethylammonium tetraphenylborate, tri-n-butylammonium tetraphenylborate, trimethylammonium tetraphenylborate, tetraethylammonium tetraphenylborate, methyl(tri-n-butyl)ammonium tetraphenylborate, benzyl(tri-n-butyl)ammonium

tetraphenylborate, dimethyldiphenylammonium tetraphenylborate, triphenyl(methyl)ammonium tetraphenylborate, trimethylanilinium tetraphenylborate, methylpyridinium tetraphenylborate, benzylpyridinium tetraphenylborate, methyl(2-cyanopyridinium) tetraphenylborate, triethylammonium tetrakis(pentafluorophenyl)borate, tri-n-butylammonium tetrakis(pentafluorophenyl)borate, triphenylammonium tetrakis(pentafluorophenyl)borate, tetra-n-butylammonium tetrakis(pentafluorophenyl)borate, tetraethylammonium tetrakis(pentafluorophenyl)borate, benzyl(tri-n-butyl)ammonium tetrakis(pentafluorophenyl)borate, methyldiphenylammonium tetrakis(pentafluorophenyl)borate, triphenyl(methyl)ammonium tetrakis(pentafluorophenyl)borate, methylanilinium tetrakis(pentafluorophenyl)borate, dimethylanilinium tetrakis(pentafluorophenyl)borate, trimethylanilinium tetrakis(pentafluorophenyl)borate, methylpyridinium tetrakis(pentafluorophenyl)borate, benzylpyridinium tetrakis(pentafluorophenyl)borate, methyl(2-cyanopyridinium) tetrakis(pentafluorophenyl)borate, benzyl(2-cyanopyridinium) tetrakis(pentafluorophenyl)borate, methyl(4-cyanopyridinium) tetrakis(pentafluorophenyl)borate, triphenylphosphonium tetrakis(pentafluorophenyl)borate, dimethylanilinium tetrakis(bis(3,5-ditrifluoromethyl)phenyl)borate, ferrocenium tetraphenylborate, silver tetraphenylborate, trityl tetraphenylborate, tetraphenylporphyrin manganese tetraphenylborate, ferrocenium tetrakis(pentafluorophenyl)borate, (1,1'-dimethylferrocenium) tetrakis(pentafluorophenyl)borate, decamethylferrocenium tetrakis(pentafluorophenyl)borate, silver tetrakis(pentafluorophenyl)borate, trityl tetrakis(pentafluorophenyl)borate, lithium tetrakis(pentafluorophenyl)borate, sodium tetrakis(pentafluorophenyl)borate, tetraphenylporphyrin manganese tetrakis(pentafluorophenyl)borate, silver tetrafluoroborate, silver hexafluorophosphate, silver hexafluoro arsenate, silver perchlorate, silver trifluoroacetate and silver trifluoromethanesulfonate.

The component (B-1) may be used solely or as a combination of plural kinds thereof.

Examples of the aluminoxane as the component (B-2) include a linear aluminoxane represented by the following formula (VII):

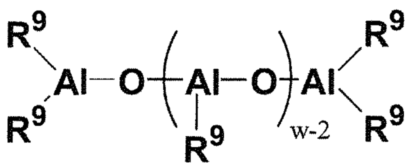

(VII)

wherein $R^9$ represents a hydrocarbon group, such as an alkyl group, an alkenyl group, an aryl group and an arylalkyl group, having from 1 to 20 carbon atoms, and preferably from 1 to 12 carbon atoms, or a halogen atom, and w represents a number of generally from 2 to 50, and preferably from 2 to 40, which is an average polymerization degree, in which groups represented by $R^9$ may be the same or different, and a cyclic aluminoxane represented by the following formula (VIII):

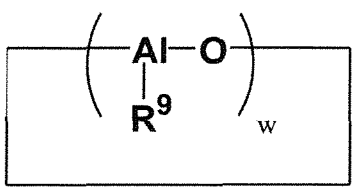

(VIII)

wherein $R^9$ and w have the same meanings as in the formula (VII).

Examples of a method for producing the aluminoxane include a method of making an alkylaluminum and a condensing agent, such as water, into contact with each other, but the specific measures therefor are not particularly limited, and they may be reacted according to a known manner.

Examples of the measures include (1) a method, in which an organoaluminum compound is dissolved in an organic solvent, and is then made into contact with water, (2) a method, in which an organoaluminum compound is added in the initial stage of polymerization, and water is added later, (3) a method, in which crystal water contained in a metal salt or the like or adsorbed water to an inorganic material or an organic material is reacted with an organoaluminum compound, and (4) a method, in which a tetraalkyldialuminoxane is reacted with a trialkylaluminum and then further reacted with water. The aluminoxane may be one that is insoluble in toluene. The aluminoxane may be used solely or as a combination of two or more kinds thereof.

In the case where the compound (B-1) is used as the catalyst component (B), the ratio of the catalyst component (A) and the catalyst component (B) used is preferably from 10/1 to 1/100, and more preferably from 2/1 to 1/10, by mol. When the ratio is outside the range, the catalyst cost per unit mass of the polymer may be increased, which may not be practical. In the case where the compound (B-2) is used, the ratio is preferably 1/1 to 1/1,000,000, and more preferably from 1/10 to 1/10,000, by mol. When the ratio is outside the range, the catalyst cost per unit mass of the polymer may

be increased, which may not be practical. The catalyst component (B) may be the compound (B-1) or (B-2) solely or may be a combination of plural kinds thereof. The polymerization catalyst used for producing the α-olefin oligomer of the present invention may contain, in addition to the components (A) and (B), an organoaluminum compound as a component (C). Examples of the organoaluminum compound as the component (C) include a compound represented by the following formula (IX):

$$R^{10}{}_{v}AlJ_{3-v} \qquad (IX)$$

wherein $R^{10}$ represents an alkyl group having from 1 to 10 carbon atoms, J represents a hydrogen atom, an alkoxy group having from 1 to 20 carbon atoms, an aryl group having from 6 to 20 carbon atoms or a halogen atom, and v represents an integer of from 1 to 3.

Specific examples of the compound represented by the formula (IX) include trimethylaluminum, triethylaluminum, triisopropylaluminum, triisobutylaluminum, dimethylaluminum chloride, diethylaluminum chloride, methylaluminum dichloride, ethylaluminum dichloride, dimethylaluminum fluoride, diisobutylaluminum hydride, diethylaluminum hydride and ethylaluminum sesquichloride. Among these, an organoaluminum compound having a hydrocarbon group having 4 or more carbon atoms bonded thereto is preferred since it is excellent in high temperature stability, and a hydrocarbon group having from 4 to 8 carbon atoms is more preferred from the same standpoint. In the case where the reaction temperature is 100°C or more, a hydrocarbon group having from 6 to 8 carbon atoms is more preferred. The organoaluminum compound may be used solely or as a combination of two or more kinds thereof.

The ratio of the catalyst component (A) and the catalyst component (C) used is preferably from 1/1 to 1/10,000, more preferably from 1/5 to 1/2,000, and further preferably from 1/10 to 1/1,000, by mol. The use of the catalyst component (C) enhances the polymerization activity per the transition metal, but an excessive amount of the catalyst component (C) is not preferred since the organoaluminum compound may be wasted and may remain in the α-olefin oligomer in a large amount.

In the production of the α-olefin oligomer of the present invention, at least one of the catalyst components may be used after supporting on a suitable carrier. The kind of the carrier is not particularly limited. An inorganic oxide carrier and other inorganic and organic carriers may be used, and an inorganic oxide carrier and other inorganic carriers are preferred.

Specific examples of the inorganic oxide carrier include $SiO_2$, $Al_2O_3$, MgO, $ZrO_2$, $TiO_2$, $Fe_2O_3$, $B_2O_3$, CaO, ZnO, BaO, $ThO_2$, and mixtures thereof, such as silica-alumina, zeolite, ferrite and glass fibers. Among these, $SiO_2$ and $Al_2O_3$ are particularly preferred. The inorganic oxide carrier may contain small amounts of a carbonate salt, a nitrate salt, a sulfate salt and the like. Examples of the carriers other than above include a magnesium compound represented by the general formula $MgR^{11}{}_{x}X^{1}{}_{y}$, examples of which include $MgCl_2$ and $Mg(OC_2H_5)_2$, and a complex salt thereof. In the general formula, $R^{11}$ represents an alkyl group having from 1 to 20 carbon atoms, an alkoxy group having from 1 to 20 carbon atoms or an aryl group having from 6 to 20 carbon atoms, $X^1$ represents a halogen atom or an alkyl group having from 1 to 20 carbon atoms, x represents 0 to 2, and y represents 0 to 2, provided that x + y = 2. The plural groups of $R^{11}$ and $X^1$ may be the same as or different from each other.

Examples of the organic carrier include a polymer, such as polystyrene, a styrene-divinylbenzene copolymer, polyethylene, poly-1-butene, substituted polystyrene and polyarylate, and starch and carbon. The carrier for the catalyst used for producing the α-olefin oligomer of the present invention is preferably $MgCl_2$, $MgCl(OC_2H_5)$, $Mg(OC_2H_5)_2$, $SiO_2$, $Al_2O_3$ or the like. While the properties of the carrier may vary depending on the kind and production method thereof, the average particle diameter thereof is generally from 1 to 300 μm, preferably from 10 to 200 μm, and more preferably from 20 to 100 μm. When the particle diameter is too small, the amount of fine powder in the polymer may be increased, and when the particle diameter is too large, the amount of coarse particles in the polymer may be increased, which may cause decrease of the bulk density and clogging of the hopper. The specific surface area of the carrier is generally from 1 to 1,000 $m^2/g$, and preferably from 50 to 500 $m^2/g$, and the pore volume thereof is generally from 0.1 to 5 $cm^3/g$, and preferably from 0.3 to 3 $cm^3/g$. When one of the specific surface area and the pore volume is outside the range, the catalyst activity may be decreased in some cases. The specific surface area and the pore volume may be obtained, for example, from the volume of nitrogen gas adsorbed according to the BET method (see, for example, J. Am. Chem. Soc., 60, 309 (1983)).

In the case where the carrier is the inorganic oxide carrier, the carrier is preferably used after baking at a temperature of generally from 150 to 1,000°C, and preferably from 200 to 800°C. In the case where at least one of the catalyst components is supported on the carrier, at least one of the catalyst component (A) and the catalyst component (B) is preferably supported, and both the catalyst component (A) and the catalyst component (B) are more preferably supported. The method for supporting at least one of the catalyst component (A) and the catalyst component (B) on the carrier is not particularly limited, and examples of the method include (1) a method of mixing at least one of the component (A) and the component (B) with the carrier, (2) a method of treating the carrier with an organoaluminum compound or a halogen-containing silicon compound, and then mixing at least one of the component (A) and the component (B) with

the treated carrier in an inert solvent, (3) a method of reacting the carrier, the component (A) and/or the component (B), and an organoaluminum compound or a halogen-containing silicon compound, (4) a method of supporting the component (A) or the component (B) on the carrier, and then mixing the carrier with the component (B) or the component (A), (5) a method of mixing a contact reaction product of the component (A) and the component (B) with the carrier, and (6) a method of making the carrier present in contact reaction of the component (A) and the component (B). In the methods (4), (5) and (6), an organoaluminum compound as the component (C) may be added.

The catalyst thus obtained may be used for polymerization after removal of the solvent for solidification, or may be used as it is for polymerization. In the production of the α-olefin oligomer of the present invention, the operation of supporting at least one of the component (A) and the component (B) on the carrier may be performed to produce the catalyst in the polymerization system. For example, such a method may be employed that at least one of the component (A) and the component (B), the carrier, and depending on necessity the organoaluminum compound as the component (C), are added to the polymerization system, and then an olefin, such as ethylene, is added under the atmospheric pressure to 2 MPa (gauge) and preliminarily polymerized at a temperature of from -20 to 200°C for approximately from 1 minute to 2 hours, thereby forming catalyst particles.

The ratio of the component (B-1) and the carrier in the catalyst used for producing the α-olefin oligomer of the present invention is preferably from 1/5 to 1/10,000, and more preferably from 1/10 to 1/500, by mass, and the ratio of the component (B-2) and the carrier is preferably from 1/0.5 to 1/1,000, and more preferably from 1/1 to 1/50, by mass. In the case where two or more kinds of the component (B) are used as a mixture, the ratios of each of the component (B) and the carrier are preferably in the aforementioned range. The ratio of the component (A) and the carrier is preferably from 1/5 to 1/10,000, and more preferably from 1/10 to 1/500, by mass. When the ratio of the component (B) (i.e. , the component (B-1) or the component (B-2) and the carrier, or the ratio of the component (A) and the carrier is outside the range, the activity may be decreased in some cases. The average particle diameter of the polymerization catalyst thus produced is generally from 2 to 200 μm, preferably from 10 to 150 μm, and particularly preferably from 20 to 100 μm, and the specific surface area thereof is generally from 20 to 1,000 $m^2/g$, and preferably from 50 to 500 $m^2/g$. When the average particle diameter is less than 2 μm, the amount of fine powder in the polymer may be increased in some cases, and when the average particle diameter exceeds 200 μm, the amount of coarse particles in the polymer may be increased in some cases. When the specific surface area is less than 20 $m^2/g$, the activity may be decreased in some cases, and when the specific surface area exceeds 1,000 $m^2/g$, the bulk density of the polymer may be decreased in some cases. In the catalyst used for producing the α-olefin oligomer, the transition metal amount per 100 g of the carrier is generally from 0.05 to 10 g, and preferably from 0.1 to 2 g. When the transition metal amount is outside the range, the activity may be decreased in some cases. By using the carrier for supporting the catalyst components, such a polymer may be obtained that has a high bulk density and an excellent particle diameter distribution, which are industrially advantageous.

In the method for producing an α-olefin oligomer of the present invention, while the polymerization method is not particularly limited, any one of a slurry polymerization method, a gas phase polymerization method, a bulk polymerization method, a solution polymerization method, a suspension polymerization method and the like may be employed, and a slurry polymerization method and a solution polymerization method are particularly preferred. As for the polymerization condition, the polymerization temperature is generally from 0 to 200°C, more preferably from 20 to 200°C, and particularly preferably from 100 to 200°C. The ratio of the raw materials to be reacted and the catalyst, i.e., the ratio of the raw material monomer/the component (A) (by mol), is preferably from 1 to 108, and particularly preferably from 100 to 105.

The viscosity of the α-olefin oligomer may be controlled by the reaction temperature, or may also be controlled by thermal decomposition of an α-olefin oligomer with high viscosity having been synthesized in advance, and decomposition of a peroxide compound.

The polymerization time is generally from 5 minutes to 30 hours, and preferably from 15 minutes to 25 hours. The hydrogen pressure is preferably from the atmospheric pressure to 10 MPa (gauge), preferably from the atmospheric pressure to 5.0 MPa (gauge), and more preferably from the atmospheric pressure to 1.0 MPa (gauge). In the method for producing an α-olefin oligomer of the present invention, the addition of hydrogen is preferred since it largely enhances the polymerization activity. A larger amount of hydrogen added enhances largely the polymerization activity, and a hydrogen pressure exceeding 10 MPa (G) causes no further influence on the activity, but disadvantageously enlarges the production equipments.

In the case where a polymerization solvent is used, examples of the solvent used include an aromatic hydrocarbon, such as benzene, toluene, xylene, ethylbenzene and decalin, an alicyclic hydrocarbon, such as cyclopentane, cyclohexane and methylcyclohexane, an aliphatic hydrocarbon, such as pentane, hexane, heptane and octane, and a halogenated hydrocarbon, such as chloroform and dichloromethane. The solvent may be used solely or as a combination of two or more kinds thereof. Depending on the polymerization method, the polymerization may be performed in the absence of a solvent.

Upon polymerization, preliminary polymerization may be performed for preparing the polymerization catalyst. The preliminary polymerization may be performed by making the catalyst component into contact with a small amount

of an olefin, but the method therefor is not particularly limited, and a known method may be employed. The olefin used for the preliminary polymerization is not particularly limited. Examples of the olefin include (D) an α-olefin having from 3 to 18 carbon atoms and mixtures thereof, and the same olefin as used in the polymerization is advantageously used. Specific examples of the preliminary polymerization include preparation of the polymerization catalyst through contact of the component (A), the component (B) and (D) an α-olefin having from 3 to 18 carbon atoms, and preparation of the polymerization catalyst through contact of the component (A), the component (B), the component (C) and an (D) α-olefin having from 3 to 18 carbon atoms. The preliminary polymerization temperature is generally from -20 to 200°C, preferably from -10 to 130°C, and more preferably from 0 to 80°C. In the preliminary polymerization, a aliphatic hydrocarbon, an aromatic hydrocarbon, a monomer and the like may be used as a solvent. The preliminary polymerization may be performed in the absence of a solvent. In the preliminary polymerization, the conditions are preferably controlled in such a manner that the limiting viscosity (η) of the preliminary polymerization product (measured in decalin at 135°C) is 0.1 dL/g or more, and the amount of the preliminary polymerization product is from 1 to 10,000 g, and particularly from 10 to 1,000 g, per 1 mmol of the transition metal in the catalyst.

A method of changing the properties of the α-olefin oligomer includes selection of the kinds and amounts of the catalyst components used and the polymerization temperature, and polymerization in the presence of hydrogen. An inert gas, such as nitrogen, may be present in the polymerization. In the production method of the present invention, there are a tendency that the reaction performed at a higher temperature provides a smaller polymerization degree, and a tendency that the use of a monomer having a smaller carbon number provides a larger polymerization degree.

[Example]

The present invention will be described in more detail with reference to examples below, but the present invention is not limited to the examples. The measurement methods and the measurement equipments for the properties are described below.

n-Mer Component Amount

0.05 g of a specimen was dissolved in 5 mL of toluene and subjected to gas chromatography (GC) measurement, thereby obtaining the n-mer component amount in terms of mass proportion.

GC Measurement Condition

column: HT-SIMDISTCB (5 m x 0. 53 mm in diameter, film thickness: 0.17 μm)
column temperature: 50°C (0.1 min), increased to 430°C at 20°C/min, 430°C (15 min)
injection (COC) temperature: oven track
detector (FID) temperature: 440°C
carrier gas: He
linear velocity: 40 cm/sec
mode: constant flow
injection amount: 0.5 μL

The molar proportion of the n-mer component amount was calculated from the mass ratio of the n-mer component amount thus obtained.

Chain Propagation Probability α

The chain propagation probability α was calculated based on the dimer component proportion C2 (% by mass) according to the following expression.

$$\text{(dimer component proportion C2)} = \frac{2}{\sum_{n=2}^{\infty} n\,\alpha^{(n-2)}} \qquad (\text{I}')$$

Kinematic viscosity and Viscosity Index

The kinematic viscosity was measured according to JIS K2283. The viscosity index was calculated from the kinematic viscosity according to JIS K2283.

Weight Average Molecular Weight (Mw) and Molecular Weight Distribution (Mw/Mn)

The weight average molecular weight and the molecular weight distribution were measured by a gel permeation chromatography (GPC) method (polystyrene conversion).
GPC Measurement Apparatus
column: TOSO GMHHR-H (S) HT
detector: IR detector for liquid chromatography, Waters 150C
Measurement Conditions
solvent: 1,2,4-trichlorobenzene
measurement temperature: 145°C
flow rate: 1.0 mL/min
sample concentration: 2.2 mg/mL
injection amount: 160 μL
calibration curve: Universal Calibration
analysis software: HT-GPC (ver. 1.0)

Meso Triad Fraction [mm]

The meso triad fraction [mm] was obtained by $^{13}$C-NMR according to the method disclosed in Macromolecules, 24, 2334 (1991) and Polymer, 30, 1350 (1989).

Amount of Vinylidene Group

The number of a terminal vinylidene group was obtained by measurement of $^1$H-NMR according to an ordinary method as follows. The content of a vinylidene group (C) (% by mol) was calculated based on the ratio of the side chain methyl group appearing at $\delta$ 0.8-1.0 and the vinylidene group appearing at $\delta$ 4.8-4.6 (2H) obtained by the $^1$H-NMR measurement. The number of a vinylidene group per one molecule was calculated from the number average molecular weight obtained by gel permeation chromatography (GPC) and the molecular weight of the monomer (M) according to the following expression.

$$\text{number of terminal vinylidene group per one molecule} = (Mn/M) \times (C/100)$$

Example 1

Reaction by preliminary activation at 50°C and hydrogen pressure of 0.15 MPa

Toluene (13 mL) and 1-decene (2 mL, 1.5 g) having been sufficiently bubbled with nitrogen were placed under a nitrogen stream into a 50-mL Schlenk flask having a stirring bar placed therein. Thereafter, triisobutylaluminum (0.4 mmol, 0.2 mL of a solution obtained by diluting to 2 mmol/L with toluene), dimethylanilinium tetrakis (pentaf luorophenyl) borate (60 μmol, 3 mL of a solution obtained by vacuum drying and then dissolving to 20 μmol/mL with toluene) and (1,1'-ethylene) (2,2'-dimethylsilylene) bis (indenyl) zirconium dichloride (40 μmol, 2 mL of a solution obtained by dissolving to 20 μmol/mL with heptane) were sequentially added thereto. The mixture was stirred for 6 hours, thereby providing a preliminary activated catalyst in the form of a solution (concentration of preliminary activated catalyst: 2 μmol/mL).
400 mL of 1-decene, 1 mmol of triisobutylaluminum and the preliminary activated catalyst (1 mL, Zr: 2 μmol) were placed in a 1-L autoclave having been dried under heat, and 0.15 MPa of hydrogen was further introduced thereto. The polymerization reaction was performed under stirring at 50°C for 5 hours, and the resulting reaction solution was decalcified with a saturated sodium hydrogen carbonate aqueous solution and then dehydrated with magnesium sulfate. The resulting $\alpha$-olefin oligomer was subjected to measurement of GC, and then after removing the unreacted monomer component by distillation, the $\alpha$-olefin oligomer was subjected to measurement of $^1$H-NMR, $^{13}$C-NMR, GPC and viscosity.

Example 2

Sequential reaction at 50°C and hydrogen pressure of 0.15 MPa

400 mL of 1-decene, 1 mmol of triisobutylaluminum, 2 μmol of (1,1'-ethylene) (2,2'-dimethylsilylene) bis(indenyl)zirconium dichloride and 8 μmol of dimethylanilinium tetrakispentafluorophenylborate were placed in a 1-L autoclave having been dried under heat, and 0.15 MPa of hydrogen was further introduced thereto. The polymerization reaction was performed under stirring at 50°C for 5 hours, thereby providing an α-olefin oligomer. The resulting reaction solution was processed and measured for properties in the same manner as in Example 1.

Example 3

Sequential reaction at 90°C and hydrogen pressure of 0.15 MPa

400 mL of 1-decene, 1 mmol of triisobutylaluminum, 2 μmol of (1,1'-ethylene) (2,2'-dimethylsilylene) bis(indenyl)zirconium dichloride and 8 μmol of dimethylanilinium tetrakispentafluorophenylborate were placed in a 1-L autoclave having been dried under heat, and 0.15 MPa of hydrogen was further introduced thereto. The polymerization reaction was performed under stirring at 90°C for 5 hours, thereby providing an α-olefin oligomer. The resulting reaction solution was processed and measured for properties in the same manner as in Example 1.

Example 4

Sequential reaction at 120°C and hydrogen pressure of 0.15 MPa

400 mL of 1-decene, 1 mmol of triisobutylaluminum, 2 μmol of (1,1'-ethylene) (2,2'-dimethylsilylene) bis (indenyl) zirconium dichloride and 8 μmol of dimethylanilinium tetrakispentafluorophenylborate were placed in a 1-L autoclave having been dried under heat, and 0.15 MPa of hydrogen was further introduced thereto. The polymerization reaction was performed under stirring at 120°C for 5 hours, thereby providing an α-olefin oligomer. The resulting reaction solution was processed and measured for properties in the same manner as in Example 1.

Example 5

Sequential reaction at 50°C and hydrogen pressure of 0.05 MPa

400 mL of 1-decene, 1 mmol of triisobutylaluminum, 2 μmol of (1,1'-ethylene) (2,2'-dimethylsilylene) bis(indenyl)zirconium dichloride and 8 μmol of dimethylanilinium tetrakispentafluorophenylborate were placed in a 1-L autoclave having been dried under heat, and 0.05 MPa of hydrogen was further introduced thereto. The polymerization reaction was performed under stirring at 50°C for 5 hours, thereby providing an α-olefin oligomer. The resulting reaction solution was processed and measured for properties in the same manner as in Example 1.

Example 6

Sequential reaction at 50°C and hydrogen pressure of 0.30 MPa

400 mL of 1-decene, 1 mmol of triisobutylaluminum, 2 μmol of (1,1'-ethylene) (2,2'-dimethylsilylene) bis (indenyl) zirconium dichloride and 8 μmol of dimethylanilinium tetrakispentafluorophenylborate were placed in a 1-L autoclave having been dried under heat, and 0.30 MPa of hydrogen was further introduced thereto. The polymerization reaction was performed under stirring at 50°C for 5 hours, thereby providing an α-olefin oligomer. The resulting reaction solution was processed and measured for properties in the same manner as in Example 1.

Comparative Example 1

Sequential reaction at 80°C and hydrogen pressure of 0.15 MPa

400 mL of 1-decene, 1 mmol of triisobutylaluminum, 2 μmol of (1,1'-methylene) (2,2'-dimethylsilylene) bis(indenyl)zirconium dichloride and 8 μmol of dimethylanilinium tetrakispentafluorophenylborate were placed in a 1-L autoclave having been dried under heat, and 0.15 MPa of hydrogen was further introduced thereto. The polymerization reaction was performed under stirring at 80°C for 5 hours, thereby providing an α-olefin oligomer. The resulting reaction

solution was processed and measured for properties in the same manner as in Example 1.

Comparative Example 2

Sequential reaction at 50°C and hydrogen pressure of 0.05 MPa

400 mL of 1-decene, 1 mmol of triisobutylaluminum, 2 μmol of (1,1'-methylene) (2,2'-dimethylsilylene) bis(indenyl)zirconium dichloride and 8 μmol of dimethylanilinium tetrakispentafluorophenylborate were placed in a 1-L autoclave having been dried under heat, and 0.05 MPa of hydrogen was further introduced thereto. The polymerization reaction was performed under stirring at 50°C for 5 hours, thereby providing an α-olefin oligomer. The resulting reaction solution was processed and measured for properties in the same manner as in Example 1.

The properties of the α-olefin oligomers obtained in Examples and Comparative Examples are shown in Tables 1-1 and 1-2 below.

Table 1-1

| | | Example | | | | | | Comparative Example | |
|---|---|---|---|---|---|---|---|---|---|
| | | 1 | 2 | 3 | 4 | 5 | 6 | 1 | 2 |
| Reaction condition | Component (B) amount (μmol) | 3 | 8 | 8 | 8 | 8 | 8 | 8 | 8 |
| | Polymerization temperature (°C) | 50 | 50 | 90 | 120 | 50 | 50 | 50 | 50 |
| | Hydrogen pressure (MPa) | 0.15 | 0.15 | 0.15 | 0.15 | 0.05 | 0.3 | 0.15 | 0.05 |
| Conversion (% by mass) | | 72.0 | 88.0 | 98.8 | 78.0 | 86.3 | 90.5 | 72.1 | 54.7 |
| n-Mer component amount (% by mass) | C20 | 7.6 | 10.0 | 35.0 | 51.4 | 17.6 | 10.6 | 4.9 | 0.7 |
| | C30 | 17.6 | 19.5 | 33.9 | 30.8 | 19.8 | 20.3 | 5.0 | 1.2 |
| | C40 | 11.4 | 12.3 | 14.0 | 9.8 | 10.8 | 12.8 | 5.0 | 1.4 |
| | C50 | 9.9 | 10.5 | 6.6 | 3.5 | 8.3 | 10.8 | 4.7 | 1.6 |
| | C60 | 8.2 | 8.6 | 3.2 | 1.2 | 6.6 | 8.8 | 4.3 | 1.6 |
| | ≥ 70 | 45.4 | 39.1 | 7.3 | 3.3 | 36.9 | 36.7 | 76.1 | 93.5 |
| chain propagation probability α | | 0.785 | 0.750 | 0.485 | 0.345 | 0.656 | 0.742 | 0.831 | 0.95 |
| n-Mer component amount in S-F distribution (% by mass) | C20 | 7.6 | 10 | 35.0 | 51.4 | 17.6 | 10.6 | 4.9 | 0.7 |
| | C30 | 9.0 | 11.3 | 25.5 | 26.9 | 17.3 | 11.8 | 6.1 | 0.9 |
| | C40 | 9.4 | 11.3 | 16.5 | 12.5 | 15.2 | 11.7 | 6.8 | 1.2 |
| | C50 | 9.2 | 10.5 | 10.0 | 5.4 | 12.4 | 10.8 | 7.0 | 1.4 |
| | C60 | 8.7 | 9.5 | 5.8 | 2.3 | 9.8 | 9.6 | 7.0 | 1.6 |
| | ≥ 70 | 56.1 | 47.4 | 7.2 | 1.5 | 27.7 | 45.5 | 68.2 | 94.2 |

Table 1-2

| | | Example | | | | | | Comparative Example | |
|---|---|---|---|---|---|---|---|---|---|
| | | 1 | 2 | 3 | 4 | 5 | 6 | 1 | 2 |
| Properties | Kinematic viscosity at 100°C ($mm^2/s$) | 7.1 | 7.0 | 3.1 | 2.5 | 6.2 | 7.9 | 39 | 99 |
| | Meso triad fraction [mm] | 21.2 | 23.1 | 11.1 | 26.8 | 22.9 | 21.5 | 33.9 | 35.5 |
| | Number of vinylidene group per one molecule | 0.4 | 0.8 | 0.9 | 1.2 | 1.0 | 0.6 | 0.5 | 0.2 |
| | Average carbon number | 10 | 10 | 10 | 10 | 10 | 10 | 10 | 10 |
| | Weight average molecular weight (Mw) | 1,322 | 1,440 | 747 | 609 | 1,521 | 1,326 | 4,100 | 7,500 |
| | Molecular weight distribution (Mw/Mn) | 1.6 | 1.5 | 1.2 | 1.2 | 1.7 | 1.6 | 1.7 | 1.8 |
| | Viscosity index | 171 | 170 | 152 | 154 | 179 | 175 | 195 | 211 |

In Examples 1 to 6, such $\alpha$-olefin oligomers are obtained that contains a larger amount of a trimer component but a smaller amount of a hexamer or higher components, as compared to the oligomer distribution in accordance with the Schulz-Flory distribution.

[Industrial Applicability]

According to the present invention, an $\alpha$-olefin oligomer that contains a larger amount of a trimer component not in accordance with the Schulz-Flory distribution, and a method for producing the same are provided. The $\alpha$-olefin oligomer of the present invention is useful as a wax component and a lubricating oil component.

## Claims

**1.** An $\alpha$-olefin oligomer having:

(1) a trimer component proportion C3 (% by mass) that is larger than a theoretical value obtained from the S-F distribution,
(2) a hexamer component proportion C6 (% by mass) that is smaller than a theoretical value obtained from the S-F distribution,
(3) a kinematic viscosity at 100°C of 20 $mm^2/s$ or less,
(4) a meso triad fraction [mm] measured by $^{13}C$-NMR of 40% by mol of less,
(5) from 0.2 to 1.0 of a vinylidene group per one molecule, and
(6) an average carbon number of a monomer unit of from 6 to 30,

wherein the S-F distribution is calculated based on a chain propagation probability $\alpha$ calculated from a dimer component proportion C2 (% by mass).

**2.** A meso transition metal compound represented by the following formula (I):

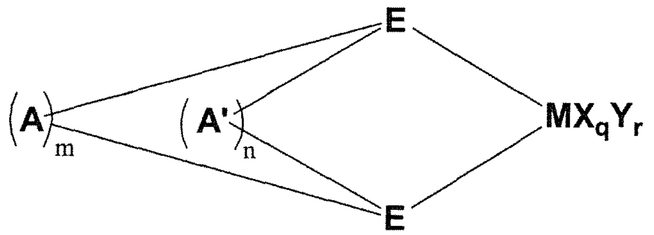

(I)

wherein in the formula (I),

M represents a metal element of Groups 3 to 10 in the Periodic Table;
X represents a σ-bonding ligand, in which when there are plural ligands represented by X, the plural ligands may be the same as or different from each other;
Y represents a Lewis base, in which when there are plural Lewis bases represented by Y, the plural Lewis bases may be the same as or different from each other;
A and A' each represent a crosslinking group selected from a hydrocarbon group having from 1 to 20 carbon atoms, a halogen-containing hydrocarbon group having from 1 to 20 carbon atoms, a silicon-containing group, a germanium-containing group and a tin-containing group, in which A and A' are different from each other;
m and n each represent an integer of 1 or more, in which m and n are different from each other, and m + n is 3 or more;
q represents an integer of from 1 to 5, which is ((valency of M) - 2);
r represents an integer of from 0 to 3; and
E represents a group represented by the following formula (II), in which two groups represented by E may be the same as or different from each other:

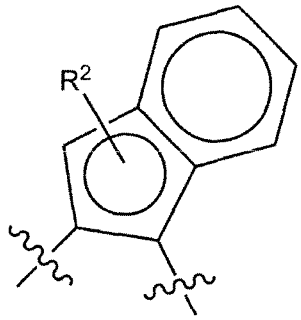

(II)

wherein in the formula (II),
$R^2$ each independently represent a group selected from a hydrogen atom, a halogen atom, a hydrocarbon group having from 1 to 20 carbon atoms, a halogen-containing hydrocarbon group having from 1 to 4 carbon atoms, a silicon-containing group and a hetero atom-containing group; and
the bonds shown with the wavy line represent bonds to $-(A)_m-$ and $-(A')_n-$.

**3.** The α-olefin oligomer according to claim 1, wherein the α-olefin oligomer is obtained with a polymerization catalyst containing:

(A) a meso transition metal compound represented by the following formula (I), and
(B) at least one component selected from (B-1) a compound that is capable of forming an ionic complex through reaction with the transition metal compound as the component (A) or a derivative thereof, and (B-2) aluminoxane:

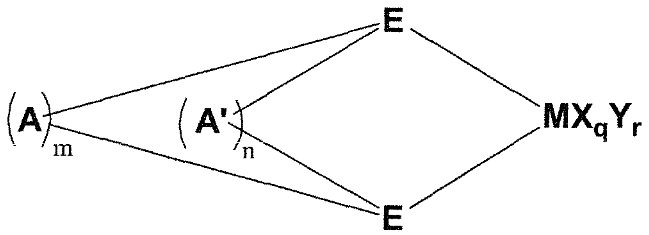

(I)

wherein in the formula (I),

M represents a metal element of Groups 3 to 10 in the Periodic Table;
X represents a σ-bonding ligand, in which when there are plural ligands represented by X, the plural ligands may be the same as or different from each other;
Y represents a Lewis base, in which when there are plural Lewis bases represented by Y, the plural Lewis bases may be the same as or different from each other;
A and A' each represent a crosslinking group selected from a hydrocarbon group having from 1 to 20 carbon atoms, a halogen-containing hydrocarbon group having from 1 to 20 carbon atoms, a silicon-containing group, a germanium-containing group and a tin-containing group, in which A and A' are different from each other;
m and n each represent an integer of 1 or more, in which m and n are different from each other, and m + n is 3 or more;
q represents an integer of from 1 to 5, which is ((valency of M) - 2);
r represents an integer of from 0 to 3; and
E represents a group represented by the following formula (II), in which two groups represented by E may be the same as or different from each other:

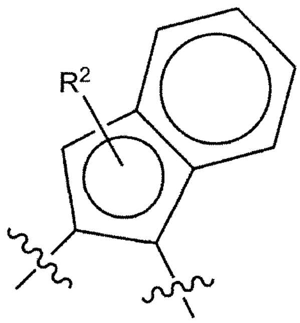

(II)

wherein in the formula (II),
$R^2$ each independently represent a group selected from a hydrogen atom, a halogen atom, a hydrocarbon group having from 1 to 20 carbon atoms, a halogen-containing hydrocarbon group having from 1 to 4 carbon atoms, a silicon-containing group and a hetero atom-containing group; and
the bonds shown with the wavy line represent bonds to $-(A)_m-$ and $-(A')_n-$.

**4.** (A) A method for producing an α-olefin oligomer, using a polymerization catalyst containing:

(A) a meso transition metal compound represented by the following formula (I), and
(B) at least one component selected from (B-1) a compound that is capable of forming an ionic complex through reaction with the transition metal compound as the component (A) or a derivative thereof, and (B-2) aluminoxane:

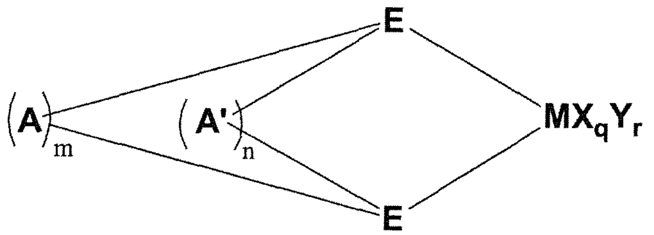

(I)

wherein in the formula (I),

M represents a metal element of Groups 3 to 10 in the Periodic Table;
X represents a σ-bonding ligand, in which when there are plural ligands represented by X, the plural ligands may be the same as or different from each other;
Y represents a Lewis base, in which when there are plural Lewis bases represented by Y, the plural Lewis bases may be the same as or different from each other;
A and A' each represent a crosslinking group selected from a hydrocarbon group having from 1 to 20 carbon atoms, a halogen-containing hydrocarbon group having from 1 to 20 carbon atoms, a silicon-containing group, a germanium-containing group and a tin-containing group, in which A and A' are different from each other;
m and n each represent an integer of 1 or more, in which m and n are different from each other, and m + n is 3 or more;
q represents an integer of from 1 to 5, which is ((valency of M) - 2);
r represents an integer of from 0 to 3; and
E represents a group represented by the following formula (II), in which two groups represented by E may be the same as or different from each other:

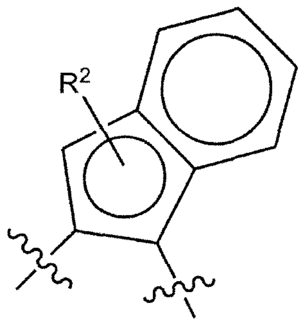

(II)

wherein in the formula (II),
$R^2$ each independently represent a group selected from a hydrogen atom, a halogen atom, a hydrocarbon group having from 1 to 20 carbon atoms, a halogen-containing hydrocarbon group having from 1 to 4 carbon atoms, a silicon-containing group and a hetero atom-containing group; and
the bonds shown with the wavy line represent bonds to $-(A)_m-$ and $-(A')_n-$.

**5.** The method for producing an α-olefin oligomer according to claim 4, wherein the polymerization catalyst is a catalyst obtained by making the component (A), the component (B) and (C) an organoaluminum compound into contact with each other in advance.

**6.** The method for producing an α-olefin oligomer according to claim 5, wherein the polymerization catalyst is a catalyst obtained by making the component (A), the component (B), the component (C) and (D) an α-olefin having from 3 to 18 into contact with each other in advance.

**7.** The method for producing an α-olefin oligomer according to any one of claims 4 to 6, wherein in the formula (I), the crosslinking group represented by $-(A)_m-$ is represented by the following formula (IV), and the crosslinking group represented by $-(A')_n-$ is represented by the following formula (IV'):

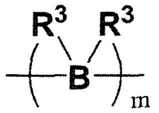 (A) (IV) 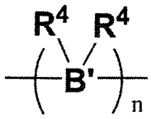 (A') (IV')

wherein in the formulae (IV) and (IV'),

B and B' each independently represent a carbon atom, a silicon atom, a germanium atom or a tin atom;
$R^3$ and $R^4$ each independently represent a hydrogen atom, an aliphatic hydrocarbon group having from 1 to 20 carbon atoms, an aromatic hydrocarbon group having from 6 to 20 carbon atoms, an oxygen-containing group having from 1 to 20 carbon atoms, an amine-containing group having from 1 to 20 carbon atoms or a halogen-containing group having from 1 to 20 carbon atoms; and
m and n each independently represent an integer of 1 or more.

**8.** The method for producing an α-olefin oligomer according to any one of claims 4 to 7, wherein the aluminoxane as the component (B-2) is a linear aluminoxane represented by the following formula (VII) and/or a cyclic aluminoxane represented by the following formula (VIII):

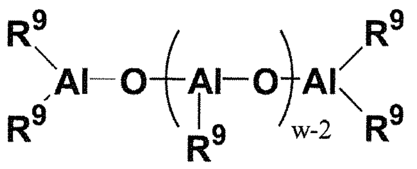 (VII)

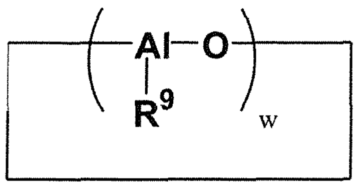 (VIII)

wherein $R^9$ represents a hydrocarbon group having from 1 to 20 carbon atoms or a halogen atom, in which groups represented by $R^9$ may be the same or different; and w represents a number of from 2 to 50, which is an average polymerization degree.

**9.** The method for producing an α-olefin oligomer according to any one of claims 4 to 8, wherein reaction is performed at a temperature of from 40 to 200°C.

**10.** The method for producing an α-olefin oligomer according to any one of claims 4 to 9, wherein reaction is performed under a hydrogen pressure of from the atmospheric pressure to 10 MPa (G).

**INTERNATIONAL SEARCH REPORT**

International application No. PCT/JP2012/069511

A. CLASSIFICATION OF SUBJECT MATTER
*C08F10/14*(2006.01)i, *C07C2/24*(2006.01)i, *C07C11/02*(2006.01)i, *C07F17/00*(2006.01)i, *C08F4/6592*(2006.01)i

According to International Patent Classification (IPC) or to both national classification and IPC

B. FIELDS SEARCHED

Minimum documentation searched (classification system followed by classification symbols)
C08F10/14, C07C2/24, C07C11/02, C07F17/00, C08F4/6592

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CA/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | WO 2010/117028 A1 (Idemitsu Kosan Co., Ltd.), 14 October 2010 (14.10.2010), claims; paragraph [0007]; examples & EP 2418228 A1 & CN 102388072 A & TW 201114779 A & KR 10-2012-0009445 A | 1,3 |
| X | JP 2001-335607 A (Idemitsu Petrochemical Co., Ltd.), 04 December 2001 (04.12.2001), claims 1, 4; paragraphs [0019], [0050] & US 2002/0143113 A1 & EP 1164146 A2 & TW 237645 B | 2,4-10 |
| X | JP 2009-57573 A (Idemitsu Kosan Co., Ltd.), 19 March 2009 (19.03.2009), claims; paragraph [0029] (Family: none) | 2,4-10 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>16 October, 2012 (16.10.12) | Date of mailing of the international search report<br>23 October, 2012 (23.10.12) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No. PCT/JP2012/069511

C (Continuation). DOCUMENTS CONSIDERED TO BE RELEVANT

| Category* | Citation of document, with indication, where appropriate, of the relevant passages | Relevant to claim No. |
|---|---|---|
| X | JP 2000-95808 A (Idemitsu Petrochemical Co., Ltd.), 04 April 2000 (04.04.2000), paragraph [0023] (Family: none) | 2 |
| X | JP 10-259207 A (Idemitsu Kosan Co., Ltd.), 29 September 1998 (29.09.1998), claim 1; paragraph [0028] & US 6316561 B1 & EP 970974 A1 & WO 1998/042757 A1 & DE 69834718 D | 2 |
| P,X | WO 2012/035710 A1 (Idemitsu Kosan Co., Ltd.), 22 March 2012 (22.03.2012), claims; paragraphs [0061] to [0063] (Family: none) | 2,4-10 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03070790 A
- JP 2001335607 A
- WO 2010053022 A
- WO 2010117028 A
- JP 2009501836 T


### Non-patent literature cited in the description


- *J. Am. Chem. Soc.,* 1940, vol. 62 (6), 1561-1565
- *Adv. Polymer. Sci,* 1974, vol. 15 (1), 1-30
- *J. Am. Chem. Soc.,* 2004, vol. 126, 10701-10712
- *J. Am. Chem. Soc.,* 1983, vol. 60, 309
- *Macromolecules,* 1991, vol. 24, 2334
- *Polymer,* 1989, vol. 30, 1350